# EUROPÄISCHE PATENTSCHRIFT

(11) **EP 1 702 680 B1**
(45) Veröffentlichungstag und Bekanntmachung des Hinweises auf die Patenterteilung: **18.11.2009**
(21) Anmeldenummer: 06115678.2
(22) Anmeldetag: 19.08.2002
(51) Int. Cl.: B01J 23/36, C07C 6/06, C07C 6/04, C07C 13/00

(54) **Olefin-Metathese an Rheniumoxid Trägerkatalysatoren**
Olefin Metathesis over supported rhenium oxide catalysts
Métathèse des oléfines utilisant des catalyseurs supportés à base d'oxyde de rhénium

(30) Priorität: 28.08.2001 DE 10142035
(43) Veröffentlichungstag der Anmeldung: 20.09.2006
(62) Teilanmeldung aus: 02018152.5
(73) Patentinhaber: Symrise GmbH & Co. KG, 37603 Holzminden (DE)
(72) Erfinder: Wöhrle, Ingo, 37603, Holzminden (DE); Reckziegel, Aurélia, 41540, Dormagen (DE); Esser, Peter, 592 Summerville, SC 29485 (US); Stürmann, Martin, 51373, Leverkusen (DE)
(74) Vertreter: Eisenführ, Speiser & Partner

(56) Entgegenhaltungen:
- DE-A- 19 727 256
- GB-A- 1 216 587
- US-A- 3 892 817
- MOL, J. C.: "Olefin metathesis over supported rhenium oxide catalysts" CATALYSIS TODAY, Bd. 51, 1999, Seiten 289-299, XP002222177
- HIETALA, J. ET AL.: "The Surface acididty of Pure and modified aluminas in Re/Al2O3 Metathesis Catalysts as Studied by 1H MAS NMR Spectroscopy and its Importance in the Ethenolysis od 1,5-Cyclooctadiene" JOURNAL OF CATALYSIS, Bd. 150, 1984, Seiten 46-55, XP002222178
- KAWAI ET AL: "Metathesis of n-alkenes over a CsNO3-Re2O7-Al2O3 catalyst" JOURNAL OF MOLECULAR CATALYSIS, ELSEVIER SEQUOIA, LAUSANNE, CH, Bd. 46, 1988, Seiten 157-172, XP002105315
- XIAODING X ET AL: "Re2O7/Al2O3.B2O3 Metathesis Catalyst" JOURNAL OF THE CHEMICAL SOCIETY, FARADAY TRANSACTIONS 1, CHEMICAL SOCIETY. LETCHWORTH, GB, Bd. 82, 1986, Seiten 1945-1953, XP002082478

## Beschreibung

Die Erfindung betrifft modifizierte Trägerkatalysatoren auf Basis von Re₂O₇/γ-Al₂O₃ zur Verwendung bei der Herstellung von Cycloalkadienen in einer Metathesereaktion, ein Verfahren zur Herstellung von Cycloalkadienen in Gegenwart dieser Trägerkatalysatoren sowie die Verwendung der hergestellten Cycloalkadiene.

Cycloalkene, insbesondere Cycloalkadiene mit einer Ringgröße zwischen 12 und 18 Kohlenstoffatomen, werden unter anderem zur Herstellung von sauerstoffhaltigen, makrocyclischen Verbindungen verwendet. Die Verbindungen können eingesetzt werden bei der Herstellung makrocyclischer Ketone, Lactone und Epoxide, die in der Duftstoffindustrie als Moschusriechstoffe begehrt sind.

Aus der EP-A 182 333 ist bekannt, dass bei Verwendung des Katalysatorsystems Re₂O₇/γ-Al₂O₃/SnR₄, wobei R für einen Alkylrest steht, durch Metathesereaktion hochverdünnter Cycloolefinlösungen in Flüssigphase die entsprechenden Cycloalkadiene erhalten werden können.

Die Herstellung von Cycloalkadienen in Flüssigphase in Gegenwart eines Trägerkatalysators auf Basis Re₂O₇/γ-Al₂O₃ durch Metathesereaktion von Cyclooctenylenen mit einem Polymerisierungsgrad größer oder gleich drei und/oder Cycloalkamonoenen ist in EP-A 343 437 beschrieben.

In der Literatur sind viele Modifizierungen bzw. Dotierungen von Trägerkatalysatoren auf Basis Re₂O₇/γ-Al₂O₃ untersucht und beschrieben worden, um Verbesserungen dieser Katalysatoren im Hinblick auf Aktivität oder Standzeit zu erzielen. Wichtig ist ebenfalls die Selektivität in Bezug auf die zu bildenden Cycloalkadiene sowie die Gesamtselektivität bezogen auf die Gesamtheit aller gewünschten MetatheseProdukte, d.h. die Summe an gewünschten Cycloalkadienen, oligomeren und polymeren Metathese-Produkten. Nicht gewünscht sind solche Metatheseprodukte, die Folge- oder Nebenprodukten entsprechen und die nicht aus den Ausgangsmaterialien planmäßig entstehen, wie beispielsweise um ein, zwei oder drei Kohlenstoffatome verengte oder erweiterte Ringgrößen.

Aus GB-1216587 ist bekannt, dass die Imprägnierung des Aluminiumoxids mit Anionen, beispielsweise mit Phosphat-Ionen, eine Aktivitätssteigerung des Re₂O₇/γ-Al₂O₃-Metathese-Katalysators bewirken kann.

Metathese-Katalysatoren mit Cäsium-Modifizierungen sind aus Journal of Catalysis 1984, 89, 452 und Journal of Molecular Catalysis 1988, 46, 157 bekannt.

In J. Chem. Soc., Faraday Trans. 1, 1986, 82, 2707 ist der Einfluss der Behandlung von Re₂O₇/γ-Al₂O₃ -Metathese-Katalysatoren mit Säuren diskutiert.

Aufgrund der notwendigen hohen Verdünnung der in die Metathesereaktion eingesetzten Cycloolefinlösungen, ist die pro Zeiteinheit erhältliche Menge an Cycloalkadienen bisher unter wirtschaftlichen, technischen sowie industriellen Aspekten unbefriedigend.

Es bestand daher die Aufgabe Trägerkatalysatoren und Verfahren bereitzustellen, mittels derer eine größere Menge an Cycloalkadienen pro Zeiteinheit erhalten werden kann. Es ist von großem Vorteil, eine höhere Produktivität und eine höhere Raum-Zeit-Leistung in dem Metatheseverfahren zu erzielen.

Es wurde nun gefunden, dass Modifizierungen mit Phosphor, Cäsium, Mineralsäure oder Kombinationen dieser Modifizierungen eine deutliche Zunahme der Aktivität und Produktivität des Re₂O₇/γ-Al₂O₃-Tägerkatalysators bewirken können. Dies macht sich insbesondere bei höheren Raum-Zeit-Geschwindigkeiten bemerkbar, wodurch die pro Zeiteinheit erhältliche Menge an Cycloalkadienen deutlich gesteigert werden kann. Besonders vorteilhaft ist, dass mit den modifizierten Tägerkatalysatoren eine exzellente Gesamtselektivität bezogen auf die Gesamtheit aller Metathese-Produkte these-Produkte erzielt werden kann, Weiterhin wurde gefunden, dass die erfindungsgemäßen Trägerkatalysatoren eine höhere Standzeit aufweisen, wodurch innerhalb eines Trägerkatalysatorzyklus mehr Metatheseprodukte und Cycloalkadiene hergestellt werden können. Darüberhinaus weisen die Trägerkatalysatoren eine höhere Gesamtlebensdauer auf.

Die Erfindung betrifft daher Trägerkatalysatoren zur Verwendung bei der Herstellung von Cycloalkadienen in einer Metathesereaktion, enthaltend a) γ-Al₂O₃ als Trägermaterial, b) 1 bis 12 Gew.% Re₂O₇, c) 0 bis 40 Gew.% SnR₄ oder SnO₂ oder eines Gemisches dieser Zinnverbindungen, wobei R für einen Alkylrest mit 1 bis 8 Kohlenstoffatomen steht, sowie eine Cäsium-Modifizierung, wobei die Angaben in Gew. % sich jeweils auf das Gesamtkatalysatorgewicht beziehen.

Ein weiterer Gegenstand der vorliegenden Erfindung ist ein Verfahren zur Herstellung von Cycloalkadienen aus Cycloalkamonoenen, Cyclopolyenen, acyclischen Polyenen oder deren Gemische in Flüssigphase durch Metathesereaktion in Gegenwart der genannten Trägerkatalysatoren, sowie die Verwendung der erfindungsgemäß erhältlichen Cycloalkadiene zur Herstellung von Riechstoffen, insbesondere zur Herstellung makrocyclischer Riechstoffe.

Unter Metathese-Lösung wird in der vorliegenden Erfindung die Edukt-Lösung, d.h. ein Lösungsmittel enthaltend mindestens einen Kohlenwasserstoff aus der Reihe der Cycloalkamonoene, Cyclopolyene oder acyclischen Polyene, verstanden.

Sofern nicht anders angegeben beziehen sich die Angaben in Gew.% auf das Gesamtgewicht des Trägerkatalysators.

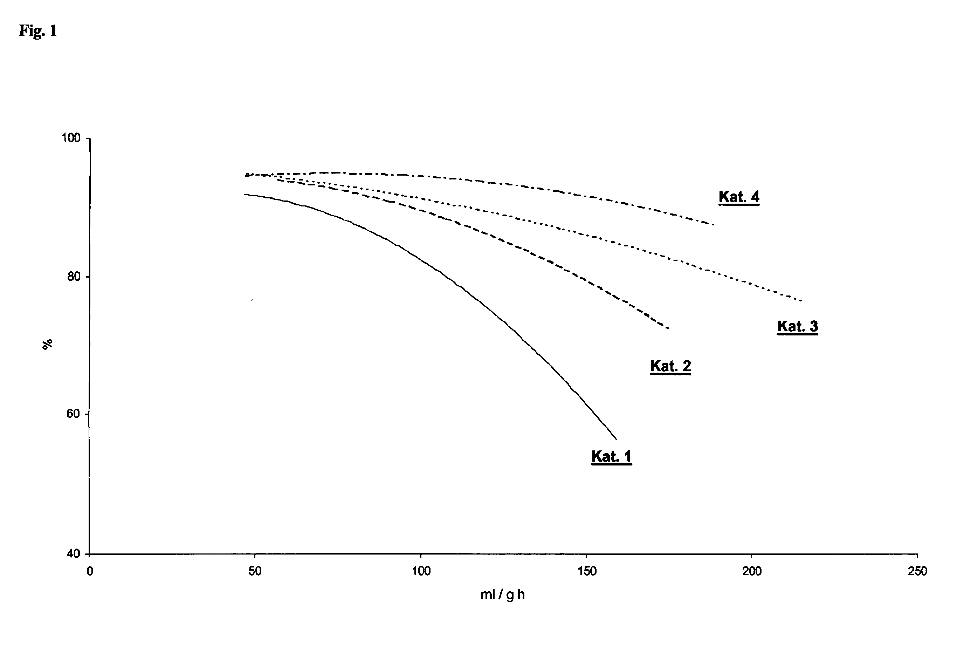

In Fig. 1 ist der Cycloocten-bezogene Umsatz (in Prozent, x-Achse) in Abhängigkeit von der Raum-Zeit-Geschwindigkeit (in ml/gh, y-Achse) in einer Metathesereaktion mit erfindungsgemäßen Trägerkatalysatoren (Kat. 2 bis 4) im Vergleich zu einem kommerziell erhältlichen Wirbelstrang-Trägerkatalysator Kat. 1 graphisch dargestellt. Die erfindungsgemäßen Trägerkatalysatoren Kat. 2 bis 4 zeigen eine deutlich höhere Aktivität, wodurch eine höhere Raum-Zeit-Leistung und eine höhere Produktivität an Cycloalkadienen erreicht werden kann. Die Trägerkatalysatoren Kat. 2 bis Kat. 4 sind in den Beispielen 1 bis 3 und die Versuchsbedingungen sind in Beispiel 4 näher erläutert.

Die im Folgenden genannten Dotierungen, Belegungen, Aufbringungen oder Behandlungen können mit üblichen, dem Fachmann bekannten Verfahren wie beispielsweise Imprägnierung oder Tränkung, auf den Trägerkatalysator aufgebracht werden.

Der Gehalt an Re₂O₇ auf dem Trägerkatalysator, bezogen auf das Trägerkatalysatorgewicht, liegt vorteilhafterweise im Bereich von 1 bis 12 Gew.-%, bevorzugt im Bereich von 2 bis 8 Gew.-%, insbesondere im Bereich von 3 bis 6 Gew.-%. Das Aufbringen des Rheniums erfolgt üblicherweise durch Imprägnierung oder Tränken des Trägermaterials mit einer wässrigen Lösung einer oder mehrerer Rheniumverbindungen und anschließender thermischer Behandlung des Guts, wobei Re₂O₇ entsteht. Geeignete Rheniumverbindungen sind beispielsweise Perrhenate wie z.B. Ammoniumperrhenat, es kann auch Perrheniumsäure oder Rheniumheptoxid selbst verwendet werden.

Es ist besonders vorteilhaft, wenn der Trägerkatalysator 0,5 bis 40 Gew.-%, bevorzugt 1 bis 20 Gew.-%, insbesondere 1 bis 10 Gew.-% SnR₄ oder SnO₂ oder eines Gemisches dieser Zinnverbindungen enthält, wobei R für einen Alkylrest mit 1 bis 8 Kohlenstoffatomen steht.

Als bevorzugte Zinntetraalkyle seien Zinntetramethyl, Zinntetraethyl, Zinntetra-n-butyl, Zinntetra-n-octyl genannt, insbesondere bevorzugt ist Zinntetramethyl. Es ist besonders vorteilhaft, wenn der Trägerkatalysator vor Beginn der Metathesereaktion mit einer Zinntetraalkyl-haltigen Lösung in Kontakt gebracht wird, wobei auch Mischungen der genannten Zinntetraalkyle eingesetzt werden können. Die Belegung mit Zinndioxid kann beispielsweise bei der Regenerierung des Zinntetraalkyl-haltigen Trägerkatalysators erfolgen, aber auch dadurch erreicht werden, dass der Trägerkatalysator mit wasserlöslichen Zinnverbindungen imprägniert oder getränkt und anschließend in sauerstoffhaltiger Atmosphäre auf 500 - 600°C erhitzt wird, wobei Zinnoxid entsteht.

In einer bevorzugten Ausfiihrungsform enthalten die erfindungsgemäßen Trägerkatalysatoren 0,1 bis 8 Gew.-%, bevorzugt 0,2 bis 5 Gew.-% insbesondere bevorzugt 0,3 bis 3 Gew.-% Phosphor bezogen auf das Gesamtgewicht des Trägerkatalysators.

Die Dotierung des Trägerkatalysators mit einer oder mehreren Phosphorverbindungen kann vor oder nach der Aufbringung des Rheniums erfolgen, bevorzugt ist die Behandlung des Trägermaterials vor der Behandlung mit einer Rheniumverbindung. Besonders geeignet ist die Behandlung mit einer wässrigen Lösung von Phosphorsäure oder Phosphaten, bevorzugt sind Ammoniumphosphate, besonders bevorzugt ist Ammoniumhydrogenphosphat.

In einer weiteren bevorzugten Ausführungsform enthalten die erfindungsgemäßen Trägerkatalysatoren 0,1 bis 6 Gew.-%, bevorzugt 0,2 bis 4 Gew.-% insbesondere bevorzugt 0,3 bis 2 Gew.-% Cäsium bezogen auf das Gesamtgewicht des Trägerkatalysators.

Die Dotierung des Trägerkatalysators mit einer oder mehreren Cäsiumverbindungen kann vor oder nach der Aufbringung des Rheniums erfolgen, bevorzugt ist die Behandlung des Trägermaterials nach der Behandlung mit einer Rheniumverbindung. Besonders geeignet ist die Behandlung mit einer wässrigen Lösung von Cäsiumsalzen, bevorzugt sind Cäsiumhalogenide, Cäsiumnitrat, Cäsiumphosphat, Cäsiumacetat, besonders bevorzugt sind Cäsiumchlorid und Cäsiumnitrat.

In einer weiteren bevorzugten Ausführungsform erfolgt eine Behandlung des Trägerkatalysators mit einer oder mehreren Mineralsäuren, wobei die Behandlung vor oder nach der Aufbringung des Rheniums erfolgen kann. Bevorzugt ist die Behandlung des γ-Al₂O₃-Trägermaterials oder des Re-beladenen Trägerkatalysators mit einer wässrigen HCl-Lösung.

Weiterhin ist es vorteilhaft, wenn die Metathesereaktion in Gegenwart eines Zinntetraalkyls durchgeführt wird. Typischerweise werden die Zinntetraalkyle vor Beginn der Metathesereaktion in die Metathese-Lösung zugegeben, und dieses Gemisch aus einem Vorratsbehältnis über das Trägerkatalysatorbett geführt. Die Zinntetraalkyle werden typischerweise in einer Menge von 0,1 bis 8 Gew.-%, bevorzugt 0,1 bis 5 Gew.-%, insbesondere 0,1 bis 2,5 Gew.-%, bezogen auf das Trägerkatalysatorgewicht, der Metathese-Lösung zugesetzt. Bevorzugte Zinntetraalkyle sind Zinntetramethyl, Zinntetraethyl, Zinntetra-n-butyl, Zinntetra-n-octyl, insbesondere bevorzugt ist Zinntetramethyl.

Die Trägerkatalysatoren weisen typischerweise spezifische Oberflächen von 100 bis 300 m²/g nach BET (Bestimmungsmethode nach Brunauer, Emmett und Teller) auf.

Die Trägerkatalysatoren werden bevorzugt als Formkörper wie beispielsweise Hohlsträngen, Extrudaten, Strangpresslingen, Kugeln, Zylindern, Würfeln, Kegeln und dergleichen eingesetzt. Besonders bevorzugt sind Kugeln, Wirbelstränge (WS) oder Zylinder.

Bevorzugt ist eine kontinuierliche Reaktionsführung, insbesondere eine vertikale Anordnung der Trägerkatalysatoren in einem Festbett, wobei vorteilhafterweise das Festbett von unten nach oben von der Metathese-Lösung durchströmt wird.

Der Gehalt in der Flüssigphase an Cycloalkamonoenen, Cyclopolyenen, acyclischen Polyenen oder deren Gemische liegt typischerweise im Bereich von 0,5 bis 10 g /1, bevorzugt im Bereich 1,0 bis 5,5 g/l, insbesondere im Bereich 2,0 bis 4,0 g/l.

Die Edukte werden in Metathese-inerten Lösungsmitteln eingesetzt. Geeignete Lösungsmittel sind beispielsweise Kohlenwasserstoffe und halogenierte Kohlenwasserstoffe, insbesondere Butan, Pentan, Hexan, Heptan, Octan, Cyclopentan, Cyclohexan, Cyclooctan, Dichlormethan, Trichlorethan. Bevorzugt sind n-Pentan, n-Hexan, n-Heptan, n-Octan, iso-Octan, Cyclopentan, Cyclohexan, insbesondere bevorzugt sind n-Pentan und n-Hexan. Es können auch Mischungen von Kohlenwasserstoffen wie z.B. Petrolether eingesetzt werden.

Vorteilhafte Cycloalkamonoene sind solche mit einer Ringgröße von 4 bis 12 Kohlenstoffatomen. Bevorzugte Cycloalkamonoene sind Cyclopenten, Cyclohexen, Cyclohepten, Cycloocten, Cyclononen, Cyclodecen und Cyclododecen. Insbesondere bevorzugt sind Cyclohepten und Cycloocten.

Besonders geeignete Cyclopolyene oder acyclische Polyene sind solche, welche aus den genannten Cycloalkamonoenen erhalten werden können. Die Cyclopolyene oder acyclischen Polyene können beispielsweise als Nebenprodukte bei metathetischen Dimerisierungen, durch ringöffnende Metathesen oder Polymerisationen gebildet werden. Im Allgemeinen weisen die Cyclopolyene und die acyclischen Polyene einen Polymerisierungsgrad von 3 bis 50, bevorzugt einen von 3 bis 20 auf. Unter dem Polymerisierungsgrad wird die Zahl der Monomereinheiten, gleich oder verschieden, verstanden, aus denen das Polyen aufgebaut ist.

Bevorzugte Cyclopolyene im Sinne der Erfindung sind Polymere bzw. Copolymere aus den genannten Cycloalkamonoenen, wobei die Cyclopolyene einen Polymerisierungsgrad von größer oder gleich 3, vorzugsweise 3 bis 50, insbesondere 3 bis 20, aufweisen. Bevorzugt werden Cyclopolyene des Cycloheptens, des Cyclooctens oder deren Copolymere verwendet.

### Besonders bevorzugte Cyclopolyene sind Cyclopolyoctenylene der Formel

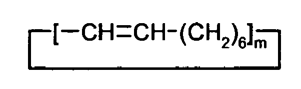

mit einem Polymerisierungsgrad m von mindestens 3, wobei m vorzugsweise im Bereich von 3 bis 50, insbesondere im Bereich von 3 bis 20 liegt.

Die Metathese-Lösungen können Cycloalkamonoene, Cyclopolyene, acyclische Polyene in beliebigen Zusammensetzungen und Mischungsverhältnissen enthalten. Bevorzugt sind Metathese-Lösungen, die Cycloalkamonoene enthalten. Werden Metathese-Lösungen verwendet, die nur Cycloalkamonoene als olefinische Verbindungen enthalten, so sind hier Cyclohepten, Cycloocten oder deren Mischungen bevorzugt. Weiterhin bevorzugt sind Mischungen aus Cycloalkamonoenen und Cyclopolyenen, wobei die Mischungen enthaltend Cyclohepten, Cycloocten oder deren Mischung und Cyclopolyheptenylen, Cyclopolyoctenylen, Copolymere aus Cyclohepten und Cycloocten oder deren Mischung besonders bevorzugt sind.

Werden Mischungen aus Cycloalkamonoenen und Cyclopolyenen eingesetzt, liegt das bevorzugte Gewichtsverhältnis im Bereich von 0,1 bis 2 zu 1, besonders bevorzugt im Verhältnis 0,2 bis 1 zu 1.

Ganz besonders bevorzugt ist eine Mischung aus Cycloocten und Cyclopolyoctenylen, wobei hier ein Verhältnis von Cycloocten zu Cyclopolyoctenylenen im Bereich 0,25 bis 0,5 zu 1 besonders bevorzugt ist.

Werden Cycloalkamonoene oder Mischungen enthaltend Cycloalkamonoene in die Metathesereaktion eingesetzt, so kann vorteilhafterweise ein Umsatz, bezogen auf den Gehalt an Cycloalkamonoenen, im Bereich von 40 bis 99 %, bevorzugt im Bereich 50 bis 95 %, insbesondere im Bereich 60 bis 85 %, eingestellt werden.

Die Metathese-Lösung kann auch geringe Anteile an Cycloalkadienen, insbesondere den zu bildenden Cycloalkadienen, d.h Produkt-Cycloalkadienen, enthalten. Diese können in geringen Mengen in den Cycloalkamonoenen, Cyclopolyenen oder den acyclischen Polyenen enthalten sein und beispielsweise destillativ bedingt sein.

Bevorzugte Cycloalkadiene, die nach dem erfindungsgemäßen Verfahren hergestellt werden können, sind bevorzugt solche mit 12 bis 18 Kohlenstoffatomen. Insbesondere bevorzugte Cycloalkadiene sind 1,8-Cyclotetradecadien, 1,8-Cyclopentadecadien und 1,9-Cyclohexadecadien. Ganz besonders bevorzugt ist 1,9-Cyclohexadecadien.

Die Metathesereaktion kann bei Temperaturen im Bereich von 0 bis 100°C durchgeführt werden, bevorzugt ist eine Temperatur im Bereich 25 bis 80°C, besonders bevorzugt eine im Bereich von 35 bis 60°C.

Beim Einsatz von Lösungsmitteln, deren Siedepunkt unterhalb der Reaktionstemperatur liegt, kann auch unter Druck gearbeitet werden. Im allgemeinen kann die Metathesereaktion bei einem Druck im Bereich von 1 bis 10 bar abs. durchgeführt werden.

Die Herstellung und Dotierung der einzusetzenden Trägerkatalysatoren erfolgt mit dem Fachmann bekannten Verfahrensweisen. Sie erfolgt üblicherweise durch Imprägnierung oder Tränken des Trägermaterials mit einer wässrigen Lösung enthaltend eine Verbindung des Dotierungselementes oder des Belegungsmaterials und anschließender Trocknung und/oder thermischer Behandlung des Guts. Die thermische Behandlung des Trägerkatalysators erfolgt in einem Temperaturbereich von 200 bis 600°C, wobei eine Maximaltemperatur im Bereich von etwa 600°C einzuhalten ist.

Nach der Verwendung bei der Metathesereaktion kann der Trägerkatalysator regeneriert und erneut für die Metathesereaktion eingesetzt werden. Wie beispielsweise in EP-B1-991 467 beschrieben, kann der Trägerkatalysator aus dem Metathesereaktor entfernt werden, mit einem Metathese-inerten Lösungsmittel gewaschen und anschließend getrocknet werden. Die thermische Behandlung des Trägerkatalysators bei der Regenerierung erfolgt in einem Temperaturbereich von 200 bis 600°C, wobei eine Maximaltemperatur von etwa 600°C einzuhalten ist. Die thermische Behandlung wird in sauerstoffhaltiger Atmosphäre durchgeführt, wie beispielsweise Luft, der gegebenenfalls zusätzlich Inertgase wie Stickstoff oder Argon beigemischt werden.

### Beispiele

### Die folgenden Beispiele erläutern die Erfindung:

### Beispiel 1: Herstellung von Kat. 2 (nicht erfindungsgemäß)

250g γ-Aluminiumoxid werden in Form von Wirbelsträngen (bezogen von der Firma KataLeuna) 6 Stunden lang bei 80°C mit einer Lösung aus 25 g Diammoniumhydrogenphosphat und 11 destilliertem Wasser getränkt, filtriert, mit 11 destilliertem Wasser gewaschen, getrocknet und 18 Stunden lang bei 580°C kalziniert. Anschließend wird dieses Material mit 130 ml einer wässrigen Lösung von 17 g Ammoniumperrhenat getränkt und getrocknet. Nach einer zweistündigen Behandlung bei 500 bis 580°C im Luftstrom wurde der Katalysator weitere 2 Stunden im Stickstoffstrom bei derselben Temperatur gehalten und anschließend auf Raumtemperatur abgekühlt. Auf diese Weise wurden 267,9 g eines Trägerkatalysators (Kat. 2) erhalten, der 3,6 Gew.% Re₂O₇ und 1,1 Gew.% Phosphor enthielt.

### Beispiel 2 : Herstellung von Kat. 3

241 g γ-Aluminiumoxid wurden in Form von Wirbelsträngen (bezogen von der Firma KataLeuna) mit 125 ml Lösung von 9 g Rheniumoxid in Wasser getränkt und anschließend getrocknet Nach einer zweistündigen Behandlung bei 500 bis 580°C im Luftstrom wurde der Katalysator weitere 2 Stunden im Stickstoffstrom bei derselben Temperatur gehalten und anschließend auf Raumtemperatur abgekühlt. Nach der Tränkung mit 125 ml einer wässrigen Lösung von 1,83 g Cäsiumnitrat, wurde der Katalysator zwei Stunden bei 120°C getrocknet, gefolgt von einer zweistündigen Behandlung bei 500°C im Luftstrom und Abkühlung im Stickstoffstrom. Auf diese Weise wurden 244,4 g Trägerkatalysator hergestellt, der 3,7 Gew.% Re₂O₇ und 0,5 Gew.% Cäsium enthielt.

### Beispiel 3: Herstellung von Kalt. 4 (nicht erfindungsgemäß)

235g γ-Aluminiumoxid wurden in Form von Wirbelsträngen (bezogen von der Firma KataLeuna) mit 244 ml wässriger Lösung von 16,6 g Ammoniumperrhenat getränkt. Das Tränken erfolgte in zwei Schritten mit je 122 ml wässriger ammoniumperrhenathaltiger Lösung mit jeweils anschließender Trocknung. Nach einer weiteren Tränkung mit 122 ml einer wässrigen Lösung von 2,26 g Chlorwasserstoff wurde der Katalysator getrocknet, zwei Stunden bei 500 bis 580°C im Luftstrom und weitere 2 Stunden im Stickstoffstrom bei derselben Temperatur behandelt. Nach dem Abkühlen unter Stickstoff wurden 243,3 g Katalysator (Kat. 4) erhalten, der 3,6 Gew.% Re₂O₇ enthielt.

### Beispiel 4:

Kat. 1 ist ein kommerziell verfügbarer Katalysator und wurde von der Firma KataLeuna bezogen (Re-Gehalt: 3,6 Gew.% Re₂O₇ auf γ-Aluminiumoxid).

Die Länge der Wirbelstränge (WS) lag typischerweise im Bereich von 10 bis 19 mm.

In einen vertikal aufgebauten Rohrreaktor (Höhe: 50 cm, Durchmesser: 1,5 cm) wurden jeweils 50 g der einzelnen in den Beispielen 1 bis 3 (Kat. 2 bis Kat. 4) beschriebenen Trägerkatalysatoren sowie des kommerziell erhältlichen Katalysators Kat.1 unter Schutzgasatmosphäre (Argon) eingehüllt. Über eine Pumpe wurde eine Lösung, enthaltend 2,5 Gew.% Zinntetramethyl (bezogen auf das Trägerkatalysatorgewicht) 3 Stunden lang bei 25°C in n-Hexan von unten nach oben im Kreis über das Festbett des Trägerkatalysators geführt. Das Trägerkatalysatorbett wurde danach bei Normaldruck und bei 45°C kontinuierlich von einer Lösung enthaltend 2,4 g Cycloocten und 0,3 Gew.% Zinntetramethyl (bezogen auf das Trägerkatalysatorgewicht) pro Liter n-Hexan von unten angeströmt.

Die pro Zeiteinheit über das Trägerkatalysatorbett geführt Menge an Metathese-Lösung, d.h. die Raum-Zeit-Geschwindigkeit, wurde über die Pumpenleistung variiert.

Die Selektivität an 1,9-Cyclohexadecadien lag über den gesamten Umsatz bei 36-38 %. Die Selektivität bezogen auf 1,9-Cyclohexadecadien und Cyclopolyoctenylenen lag bei etwa 99 %.

In Fig. 1 ist der Cycloocten-bezogene Umsatz (in Prozent, x-Achse) in Abhängigkeit von der Raum-Zeit-Geschwindigkeit (in ml / gh, γ-Achse) in einer Metathesereaktion mit den Trägerkatalysatoren Kat. 2 bis 4 im Vergleich zu einem kommerziell erhältlichen Wirbelstrang-Trägerkatalysator Kat. 1 graphisch dargestellt. Die erfmdungsgemäßen Trägerkatalysatoren Kat. 2 bis 4 zeigen eine deutlich höhere Aktivität, wodurch eine höhere Raum-Zeit-Leistung und eine höhere Produktivität an Cycloalkadienen erreicht werden kann.

## Patentansprüche

1. Verwendung eines Trägerkatalysators in der Herstellung eines Cycloalkadiens in Flüssigphase durch Metathesereaktion, wobei der Trägerkatalysator umfasst:
a) γ- Al₂O₃ als Trägermaterial,
b) 1 bis 12 Gew.-% Re₂O₇
c) 0 bis 40 Gew.% SnR₄ oder snO₂ oder eines Gemisches dieser Zinnverbindungen, wobei R für ein Alkylrest mit 1 bis 8 Kohlenstoffatomen steht,
wobei der Trägerkatalysator eine Cäsium-Modifizierung enthält, und
wobei die Angaben in Gew.-% jeweils auf das Gesamtkatalysatorgewicht bezogen sind.

2. Verwendung nach Anspruch 1, **dadurch gekennzeichnet, dass** der Trägerkatalysator 0,1 bis 8 Gew.% Phosphor enthält.

3. Verwendung nach Anspruch 1, wobei der Trägerkatalysator 0,1 bis 6 Gew.-% Cäsium enthält.

4. Verwendung nach einem der Ansprüche 1 bis 3, wobei zur Durchführung der Metathesereaktion in der Flüssigphase ein Zinntetraalkyl in einer Menge von 0,1 bis 8 Gew.% bezogen auf das Trägerkatalysatorgewicht verwendet wird.

5. Verfahren zur Herstellung eines Riechstoffs, umfassend das Herstellen eines Cycloalkadiens und das Umsetzen des so hergestellten Cycloalkadiens zu einem Riechstoff, wobei das Cycloalkadien in Flüssigphase durch Metathesereaktion mit einem Trägerkatalysator hergestellt wird, der umfasst:
a) γ-Al₂O₃ als Trägermaterial,
b) 1 bis 12 Gew.-% Re₂O₇
c) 0 bis 40 Gew.-% SnR₄ oder SnO₂ oder eines Gemisches dieser Zinnverbindungen, wobei R für ein Alkylrest mit 1 bis 8 Kohlenstoffatomen steht,
wobei der Trägerkatalysator eine Cäsium-Modifizierung enthält, und
wobei die Angaben in Gew.-% jeweils auf das Gesamtkatalysatorgewicht bezogen sind.

6. Verfahren nach Anspruch 5, **dadurch gekennzeichnet, dass** der Trägerkatalysator 0,1 bis 8 Gew.-% Phosphor enthält.

7. Verfahren nach Anspruch 5, wobei der Trägerkatalysator 0,1 bis 6 Gew.-% Cäsium enthält.

8. Verfahren nach einem der Ansprüche 5 bis 7, wobei zur Durchführung der Metathesereaktion in der Flüssigphase ein Zinntetraalkyl in einer Menge von 0.1 bis 8 Gew.-% bezogen auf das Trägerkatalysatorgewicht verwendet wird.

## Claims

1. Use of a supported catalyst in the preparation of a cycloalkadiene in the liquid phase by a metathesis reaction, wherein the supported catalyst comprises:
a) γ-Al₂O₃ as the support material,
b) 1 to 12 wt.% of Re₂O₇,
c) 0 to 40 wt.% of SnR₄ or SnO₂ or of a mixture of these tin compounds, wherein R represents an alkyl radical having 1 to 8 carbon atoms,
wherein the supported catalyst contains a caesium modification, and wherein the amounts in wt.% are in each case based on the total weight of the catalyst.

2. Use according to claim 1, **characterized in that** the supported catalyst contains 0.1 to 8 wt.% of phosphorus.

3. Use according to claim 1, wherein the supported catalyst contains 0.1 to 6 wt.% of caesium.

4. Use according to one of claims 1 to 3, wherein a tin tetraalkyl in an amount of from 0.1 to 8 wt.%, based on the weight of the supported catalyst, is used for carrying out the metathesis reaction in the liquid phase.

5. Process for the preparation of an odoriferous substance, comprising preparation of a cycloalkadiene and conversion of the cycloalkadiene prepared in this way into an odoriferous substance, wherein the cycloalkadiene is prepared in the liquid phase by a metathesis reaction with a supported catalyst which comprises:
a) γ-Al₂O, as the support material,
b) 1 to 12 wt.% of Re₂O₇,
c) 0 to 40 wt.% of SnR₄ or SnO₂ or of a mixture of these tin compounds, wherein R represents an alkyl radical having 1 to 8 carbon atoms,
wherein the supported catalyst contains a caesium modification, and wherein the amounts in wt.% are in each case based on the total weight of the catalyst.

6. Process according to claim 5, **characterized in that** the supported catalyst contains 0.1 to 8 wt.% of phosphorus.

7. Process according to claim 5, wherein the supported catalyst contains 0.1 to 6 wt.% of caesium.

8. Process according to one of claims 5 to 7, wherein a tin tetraalkyl in an amount of from 0.1 to 8 wt.%, based on the weight of the supported catalyst, is used for carrying out the metathesis reaction in the liquid phase.

## Revendications

1. Utilisation d'un catalyseur supporté dans la préparation d'un cycloalcadiène en phase liquide par une réaction de métathèse, le catalyseur supporté comprenant:
a) γ-Al₂O₃ comme matériau support,
b) 1 à 12 % en poids de Re₂O₇
c) 0 à 40 % en poids de SnR₄ ou SnO₂ ou d'un mélange de ces composés étain, où R représente un radical alkyle de 1 à 8 atomes de carbone,
où le catalyseur supporté contient une modification du césium, et
où les indications en % en poids se rapportent respectivement au poids total du catalyseur.

2. Utilisation selon la revendication 1, **caractérisée en ce que** le catalyseur supporté comprend de 0,1 à 8 % en poids de phosphore.

3. Utilisation selon la revendication 1, où le catalyseur supporté comprend de 0,1 à 6 % en poids de césium.

4. Utilisation selon l'une des revendications 1 à 3, où, pour réaliser la réaction de métathèse en phase liquide, on utilise un tétraalkylétain en une quantité de 0,1 à 8 % en poids par rapport au poids du catalyseur supporté.

5. Procédé de préparation d'un parfum, comprenant la préparation d'un cycloalcadiène et la conversion du cycloalcadiène ainsi préparé en un parfum, où le cycloalcadiène est préparé par réaction de métathèse en phase liquide avec un catalyseur supporté, qui comprend :
a) γ-Al₂O₃ comme matériau support,
b) 1 à 12 % en poids de Re₂O₇
c) 0 à 40 % en poids de SnR₄ ou SnO₂ ou d'un mélange de ces composés étain, où R représente un radical alkyle de 1 à 8 atomes de carbone,
où le catalyseur supporté contient une modification du césium, et
où les indications en % en poids se rapportent respectivement au poids total du catalyseur.

6. Procédé selon la revendication 5, **caractérisé en ce que** le catalyseur supporté comprend de 0,1 à 8 % en poids de phosphore.

7. Procédé selon la revendication 5, où le catalyseur supporté comprend de 0,1 à 6 % en poids de césium.

8. Procédé selon l'une des revendications 5 à 7, où, pour réaliser la réaction de métathèse en phase liquide, on utilise un tétraalkylétain en une quantité de 0,1 à 8 % en poids par rapport au poids du catalyseur supporté.
